# EUROPEAN PATENT SPECIFICATION

(11) **EP 4 186 547 B1**
(45) Date of publication and mention of the grant of the patent: **06.08.2025**
(21) Application number: 21210362.6
(22) Date of filing: 25.11.2021
(51) Int. Cl.: A61M 15/00, A61M 16/00, G01N 33/48, G01N 33/50

(54) **IN-VITRO AIRWAYS NEBULIZATION ASSAY**
IN-VITRO-ATEMWEGSVERNEBELUNGSTEST
ANALYSE DE NÉBULISATION IN-VITRO DES VOIES RESPIRATOIRES

(43) Date of publication of application: 31.05.2023
(73) Proprietor: PKDERM, 06600 Antibes (FR)
(72) Inventor: Osman-Ponchet, Hanan, 06600 Antibes (FR); Barthe, Manon, 06600 Antibes (FR)
(74) Representative: Axe PI

(56) References cited:
- BECKER: "'P-097 Aerosol Performance of Spray Dried MMI-0100 from the Microdose Inhaler for Treatment of Pumonary Fibrosis'", JOURNAL OF AEROSOL MEDICINE AND PULMONARY DRUG DELIVERY, vol. 26, no. 2, 1 April 2013 (2013-04-01), US, pages A - 1, XP055463858, ISSN: 1941-2711, DOI: 10.1089/jamp.2013.00A1
- RÖHM MARTINA ET AL: "A comprehensive screening platform for aerosolizable protein formulations for intranasal and pulmonary drug delivery", INTERNATIONAL JOURNAL OF PHARMACEUTICS, ELSEVIER, NL, vol. 532, no. 1, 14 September 2017 (2017-09-14), pages 537 - 546, XP085206134, ISSN: 0378-5173, DOI: 10.1016/J.IJPHARM.2017.09.027

## Description

### FIELD OF THE INVENTION

The present invention is relating to the medical domain and regards to the treatment of airways pathologies and particularly on inflammation and/or inflammatory processes like SARS-coronavirus infection.

More particularly, the instant invention is relative to an *in-vitro* airways nebulization assay, comprising a nebulization system with a nebulizer apparatus and a 3D reconstructed airways epithelium. The invention provides the use of an *in-vitro* airways nebulizing assay for evaluating the effect of products or active ingredients or botanical extracts on airways pathologies and particularly on inflammation and/or inflammatory processes.

### BACKGROUND

It is known that drug aerosol therapy provides good results for bronchial asthma with dexamethasone as disclosed by Carl Arbesman et al. (J. Allergy, July-August 1963, Vol.14 Number 14) and Röhm Et AL (Int. Journal of Pharmaceutics, September 2017, Vol. 532 Number 1, pages 537-546) However, nebulization is rarely used in airways treatments.

### PROBLEM TO BE SOLVED

Therefore, there is a need for a simple, robust, cost-effective, accurate assay for testing efficiency treatment by nebulization of compounds, drugs, or any other treatment of airways pathologies and particularly on inflammation and/or inflammatory processes like SARS-coronavirus infection.

### SUMMARY OF THE INVENTION

The present invention provides a technical solution to the above-mentioned problems in providing an in-vitro airways nebulization assay and a method for assessing treatment efficacy.

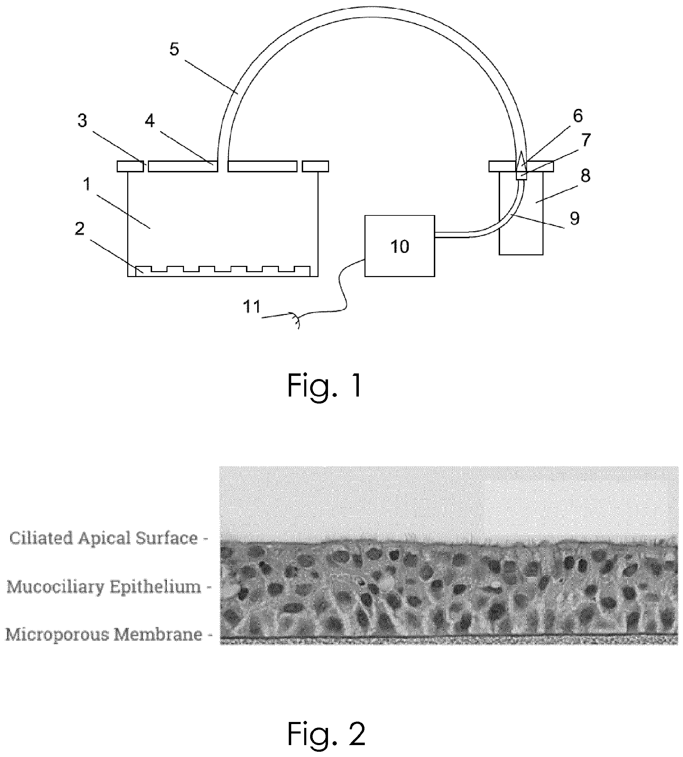

In particular, the instant invention provides an in-vitro airways nebulization assay comprising at least:
- a nebulization system as disclosed in fig. 1 with a nebulizer apparatus ; and
- a 3D reconstructed airways epithelium and the further components as required by claim 1.

As shown below, the specific interaction of the 3D airways epithelium and the nebulizer apparatus provides a unique assay for searching treatment of airways pathologies.

For the purpose of the instant invention, the 3D reconstructed airways epithelium is obtained from bronchoalveolar cell line harvested in culture medium at the air-liquid interface.

Accordingly, the nebulization system comprises at least as shown in figure 1:
- a nebulizer tank (8) ;
- a compressor (10) ;
- an air pipe (9) to propel the solution to be tested into the nozzle (7) through the vaporizer head (6) ;
- a vaporization chamber (1) and a chamber cover (4); and
- a support (2) in the chamber (1).

In the context of the present invention, the support (2) is a 6- well plate to 48- well plate, preferentially a 12-well plate to 24-well plate or any other plate compatible with nebulization.

The invention also regards the use of an *in-vitro* airways nebulizing assay as described above for evaluating products or active ingredients or botanical extracts effects on airways pathologies and particularly on inflammation and/or inflammatory processes. In the context of this invention and if not mentioned otherwise, the term products should be meant as any product and/or active ingredients and/or botanical root extracts or any molecule pharmacologically active or any chemical molecule synthetized from botanical extracts.

Accordingly, the evaluation of products (as defined above) effects is comprising the steps of:
a) taking a sample of airways tissue or using 3D reconstructed airways tissue, culturing the cells under appropriate conditions able to provide an epithelium;
b) placing a sample of airways epithelium upon a support (2) in a chamber (1);
c) switching on the nebulizing system;
d) applying a treatment by nebulization;
e) measuring the mRNA expression levels of at least one inflammatory interleukin;
f) comparing the values with standard expression levels of same inflammatory interleukin mRNA expression previously determined without treatment;
g) the modulation of inflammatory interleukin mRNA expression is provided when inflammatory interleukin mRNA expression level with product application (nebulization) is increased more than two (2) folds or decreased more than 20% in comparison with inflammatory interleukin mRNA expression level without product application and formulate a conclusion.

In the context of the invention, the sample of airways epithelium is within a medium. Preferentially, said medium of airways epithelium is initially without Hydrocortisone.

The time of treatment as disclosed in step d) should be applied according to the product and the epithelium cells targeted. The average time of application is between 5 to 30 minutes. Preferentially, this time is between 10 and 20 minutes.

Similarly, the nebulization jet flow is preferentially adjustable. An average jet flow between 4 and 7 liter per minute (I/min) is applicable. Preferably, the minimum nebulizing output is between 0.2 and 0.5 ml/min.

According to the instant invention, the at least one inflammatory interleukin is selected from: Interleukin 1 (IL-1), Interleukin 5 (IL-5), Interleukin 6 (IL-6), Interleukin 8 (IL-8), Interleukin 13 (IL-13), Interleukin 17 (IL-17) and TNF-alpha.

Indeed, Interleukin-1 (IL-1) was the first interleukin to be identified and characterized as a major endogenous pyrogen and a potent proinflammatory and pleiotropic molecule involved in resistance to microbes and in injury. Since IL-1's discovery, the IL-1 system has impressively grown and now its ligands and receptors are recognized as large and complex families of molecules involved in host responses in infections and inflammation, as well as in the activation of innate and adaptive cells. Indeed, the deregulated or excessive activation of the IL-1 system is the potential cause of dangerous and detrimental local or systemic inflammatory reactions, as well as autoimmune or allergic responses, and anti-IL-1 therapies have had a tremendous impact on inflammatory diseases.

Interleukin 5 (IL-5) is a cytokine that stimulates the production of eosinophils in asthma providing an asthma phenotyping such as eosinophil asthma (the characterization of the type of asthma).

Interleukin 6 (IL6) is a cytokine belonging to the trio of pro-inflammatory cytokines of innate immunity (interleukin-1, interleukin-6 and tumor necrosis factor) in the acute phase of inflammation (L.Velazquez-Salinas et al. « The Role of Interleukin 6 During Viral Infections », Frontiers in Microbiology, vol. 10, 2019). In particular, it stimulates the secretion of acute phase proteins during the reaction of the innate immune system (APP, or acute phase protein). Interleukin 6 is a key cytokine in the regulation of acute and chronic inflammation and acts as a messenger between cells involved in this process.

Interleukine 8 (IL-8) is produced in particular by epithelial cells following the detection of potentially pathogenic microbiological or chemical agents. Its main role is to ensure the recruitment of polynuclear neutrophils at the site of infection.

Interleukin 13 (IL-13) is a cytokine that stimulate the growth and differentiation of B lymphocytes. IL-13 is suspected to play a role in airway inflammation and bronchial remodeling in asthma (R.J Russel et al. The Lancet Respiratory Medicine, online publication 21 May 2018).

Interleukin 17 (IL-17 or IL-17A) is a cytokine that originates from the IL-17 family of cytokines. This cytokine induces the secretion of chemokines such as interleukin 8, which allow the recruitment of neutrophils 2.

TNF-alpha (TNF-a) is a potent multifunctional cytokine that plays a central role in the pathogenesis of many inflammatory diseases such as asthma and allergic rhinitis. This proinflammatory cytokine is produced by a variety of cells in the airways and is released via the IgE-dependent activation of mast cells. The elevated levels of TNF-alpha can be detected in the airways, bronchoalveolar lavage (BAL) fluid and nasal lavage from asthmatic and rhinitic patients.

Accordingly, in a preferred embodiment of the invention, the at least one inflammatory interleukin is selected from: Interleukin 6 (IL-6), Interleukin 8 (IL-8), and TNF-alpha.

Hence, in a further aspect of the invention, the evaluation of products effects provides that in step e) the mRNA expression level of at least IL-8 is measured and compare in step f) to values with standard expression levels of same IL-8 mRNA expression previously determined without treatment. Accordingly, in step g) the modulation of IL-8 mRNA expression is provided when IL-8 mRNA expression level with product application is increased more than two (2) folds or decreased more than 20% in comparison with IL-8 mRNA expression level without product administration.

In another further aspect of the invention, the evaluation of product effects provides that in step e) the inflammatory interleukin mRNA expression level of at least IL-8 is measured by selected optical density, RT-PCR or any other applicable method well known by the skilled artisan.

One embodiment of the invention provides also an *in-vitro* assay for assessing treatment of inflammation and/or inflammatory diseases comprising the steps of:
a) taking a sample of airways tissue or using 3D reconstructed airways tissue, culturing the cells under appropriate conditions able to provide at least airways cells layer;
b) placing the sample of airways tissue upon a support (2) in a chamber (1);
c) applying a treatment by nebulization by switching on the nebulizing system;
d) measuring by quantitative RT-PCR the mRNA expression levels of at least IL-8;
e) comparing the values with standard expression levels of same gene target receptors previously determined without treatment;
f) the modulation of IL-8 mRNA expression is provided when IL-8 mRNA expression level with product application is increased more than two (2) folds or decreased more than 20% in comparison with IL-8 mRNA expression level without product application and formulate a conclusion.

### BRIEF DESCRIPTION OF THE DRAWINGS

Figures described below and in the appendices are illustrative of the instant invention:
[Figure 1] illustrates the global nebulization assay.
   The general use of this nebulization assay is as follows:
   Fill in the nebulizer tank (8) with a correct amount (between 2 ml and max.10 ml) of a solution with products or active ingredients or botanical extracts to be evaluated.
   Switch on the compressor (10). Hence, pressurized air in the air pipe (9) propels the solution to be tested into the nozzle (7) through the vaporizer head (6) to be changed in vaporized droplets which achieve the vaporization chamber (1) through the pipe (5) and the chamber cover (4). The cover (4) is preferentially fitted with fitting system and at least one aperture (3).
   The support (2) in the chamber (1) is in a preferred embodiment a 6 to 48- well plate, preferentially a 12-well plate to 24-well plate but any other plate compatible with nebulization is applicable.
[Figure 2] shows Nebulization test on a bronchial model EpiAirway AIR-100. This alveolar model is prepared from bronchial / tracheal epithelial cells tissues and it mimics respiratory tissue. This in vitro bronchial model provides successive layers:
   The basal layer is made of a microporous membrane. Above said basal layer, is a mucociliary epithelium covered by a ciliated apical surface forming the last layer.
[Figure 3] shows Goblet cells secreting mucus.
[Figure 4] shows comparative IL-8 mRNA expression in the EpiAirway (compared to the control) treated with LPS 100 µg/mL or LPS 100 µg/mL + Dexamethasone (Dex) 100 µM
[Figure 5] shows comparative IL-6 mRNA expression in the EpiAirway (compared to the control) treated with LPS 100 µg/mL or LPS 100 µg/mL + Dexamethasone (Dex) 100 µM.
[Figure 6] shows comparative TNF-alpha mRNA expression in EpiAirway (versus control) treated with LPS 100 µg/mL or LPS 100 µg/mL + Dexamethasone (Dex) 100 µM
[Figure 7] shows the comparison results with the systemic and the nebulization treatment for 3 inflammation markers, IL-8, IL-6 and TNF-a, wherein:
   Figure 7 A provides comparative results for IL-8
   Figure 7 B provides comparative results for IL-6
   Figure 7 C provides comparative results for TNF- alpha
[Figure 8] illustrates the initial steps required for developing a nebulization system which will be used in the nebulization assay of the invention, wherein:
   Figure 8A provides the Tartrazine standard range with reading the absorbance at 405 nm.
   Figure 8B provides reproducibility tests with nebulization system of the invention in carrying out the test 5 times.
   Figure 8C provides an average reproducibility of tests shown in Figure 8B, between 0.1 and 0.25 for an OD at 405nm.

The following examples illustrate the invention but not be considered as a limitation of the scope of the instant invention.

### EXAMPLE 1: Development and validation of a nebulization system for use on lung tissue

This example describes the initial steps required for developing a nebulization system which will be used in the nebulization assay of the invention as it is described in the specification and the other examples.

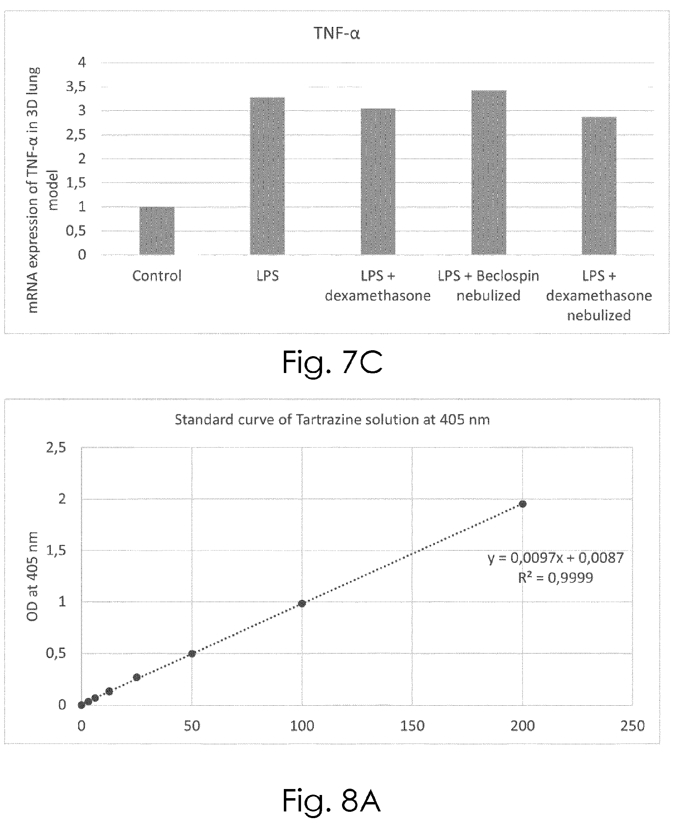

The validation of the nebulization system is initially obtained in 3 steps with a food coloring, the Tartrazine, which is a hyper soluble in water compound having a molecular weight of 534 g / mol, provided under ref. # 191891000 at Acros organics (Fisher scientific).
1^{st} step: The Tartrazine standard range as shown in figure 8A, is obtained under the following conditions to achieve a higher standard range of tartrazine ranging from 3.13 to 200 µg / mL. - Reading the absorbance at 405 nm
2^{nd} step: nebulization duration validation
   - Nebulization of a 24-well plate for 5, 10 and 20 min with the stock solution at 20 mg/mL
   - Collect the membranes and transfer them to 500 µL of water
   - 0.2 mL/min flow rate
   - Particle size < 5µm
   - Reading the absorbance at 405 nm

This part of development of the nebulization assay according to the invention provides the best nebulization duration in comparing 5 min and 10 min. Accordingly, the test is carried out according to the following conditions:
- Nebulization of a 24-well plate for 5 and 10 min (5 min nebulization, pause of 30 min then 10 min nebulization in a new plate) with the stock solution at 20 mg / mL
- Collect the membranes and transfer them to 500 µL of water
- Reading the absorbance at 405 nm
- Choice of central wells of the plate

### 3^{rd} step: reproducibility tests

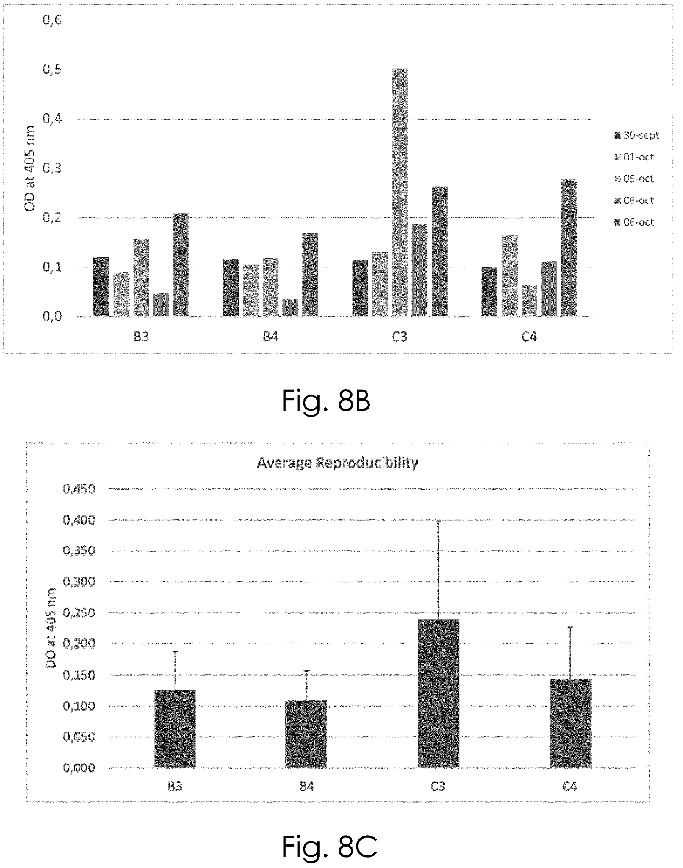

These tests have been performed 5 times over 10 min time, and the 4 central wells have been collected and absorbance (optical density - OD) had been read at 405 nm. The results as shown in Figure 8B, provide a successful reproducibility. An average reproducibility between 0.1 and 0.25 for an OD at 405nm is shown in Figure 8C.

To conclude, this example provides the guidance for developing and validating the nebulization system to be used in the nebulization assay of the invention as provided in the further examples.

### EXAMPLE 2: Validation of a nebulization model in bronchial alveolar 3D model (EpiAirway)

The present example provides the validation process of a specific nebulization model to be used as *in-vitro* airways nebulization assay according to the invention. Said validation relates to inflammation markers expression (IL8, TNF-a, IL-6) in bronchial alveolar 3D model (EpiAirway).

### Materials and methods

Nebulization test on a bronchial model:
EpiAirway AIR-100 (MatTek) are tissues from bronchial / tracheal epithelial cells and mimics respiratory tissue *in-vitro.*
Assay Conditions: 2 tissues per conditions, medium without hydrocortisone Control
Lipopolysaccharide (LPS) 100 µg / mL
LPS 100 µg / mL + Dexamethasone 100 µM
Those solutions are prepared as Systemic treatment, in the culture medium+ nebulization with physiological serum.
LPS 100 µg / mL (in culture medium) + Dexamethasone nebulized 100 µM
LPS 100 µg / mL (in culture medium) + Beclospin nebulized 100 µM
These two last nebulization solutions are prepared with physiological serum and nebulized.
Protocol: Treatment is done in culture medium. LPS and dexamethasone were added in culture medium. Treatment duration was 24 hours.

### Results:

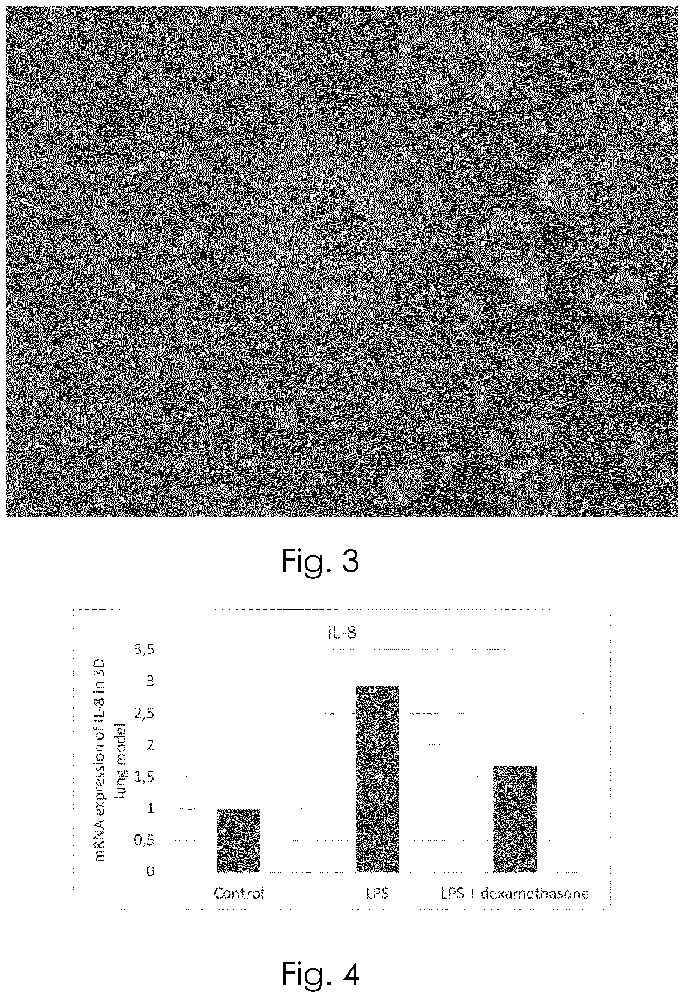

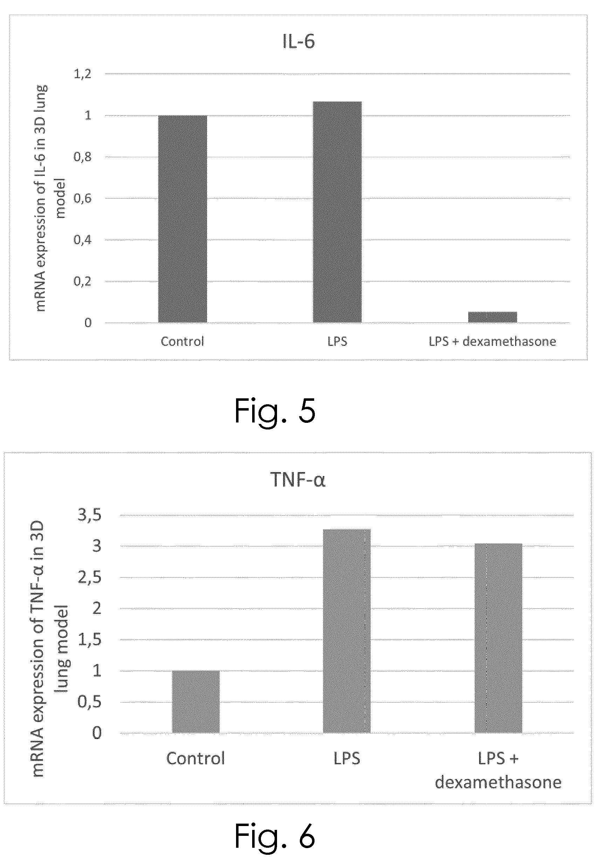

- The results are depicted in figure 4,5 and 6 and clearly show the inflammatory effect of LPS (increase of expression of IL-6, IL-8 and TNF-alpha). In addition, the results clearly show that the LPS inflammatory effect is treated with Dexamethasone.

These results confirm that model according to the instant invention is validated for demonstrating anti-inflammatory treatment efficiency.

### EXAMPLE 3: Comparison between systemic and nebulization treatment.

The present example compares the results with the systemic and the nebulization treatment for 3 inflammation markers, IL-8, IL-6 and TNF-a.

### Protocol:

The nebulization is practiced with the mounting according to figure 1 for the 10 min with a flow rate of 0.2 mL / min, the size of the nebulized particles is between 3.5 and 4.5 µm. Treatment duration lasted 24 hours.

Results: provided in table1 below:

**[table 1]**

| | | Fold change |
|---|---|---|
| Control | IL-8 | 1,00 |
| | TNF-α | 1,00 |
| | IL-6 | 1,00 |
| LPS | IL-8 | 2,93 |
| | TNF-α | 3,28 |
| | IL-6 | 1,07 |
| LPS + dexamethasone | IL-8 | 1,67 |
| | TNF-α | 3,05 |
| | IL-6 | 0,05 |
| LPS + Beclospin nebulized | IL-8 | 2,39 |
| | TNF-α | 3,42 |
| | IL-6 | 0,52 |
| LPS + dexamethasone nebulized | IL-8 | 1,92 |
| | TNF-α | 2,87 |
| | IL-6 | 0,28 |

Data in systemic treatment are provided for:
Control untreated
Lipopolysaccharide (LPS)
LPS + Dexamethasone

Data in nebulization treatment are provided for:
LPS + Beclospin
LPS+ Dexamethasone

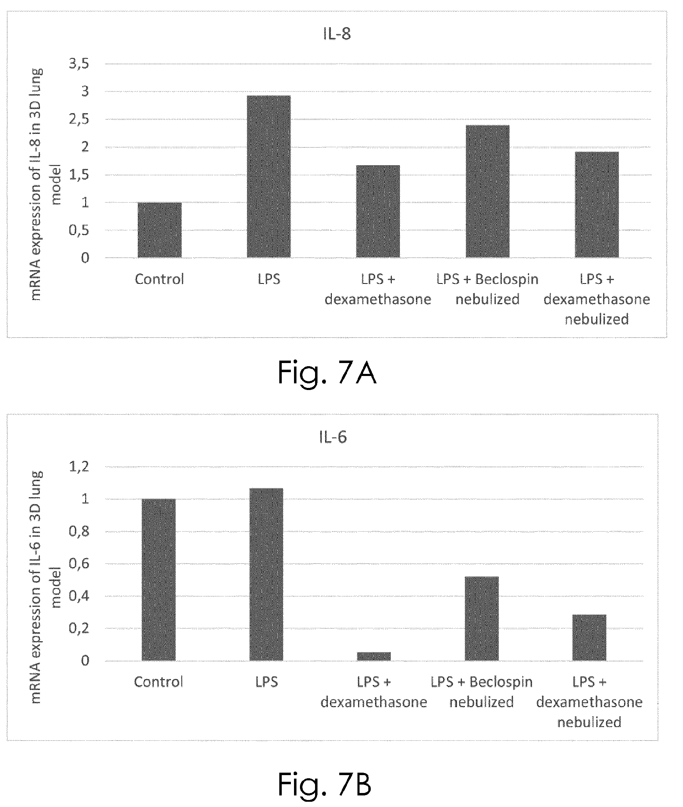

Results are depicted in figure 7:
Fig. 7 A provides results for IL-8
Fig. 7 B provides results for IL-6
Fig. 7 C provides results for TNF-alpha

Taken together these results are demonstrating that nebulization is an efficient treatment method. Indeed, this example compares the results with the systemic and the nebulization treatment and show that better results are obtained with nebulization method. This example shows in addition that the development of airways nebulization treatment assay can be used to treat several airways pathologies in targeting different cell types.

## Claims

1. An *in-vitro* airways nebulization assay comprising at least:
- a nebulization system with a nebulizer apparatus; and
- a 3D reconstructed airways epithelium.
wherein nebulization system comprises at least:
- a nebulizer tank (8) ;
- a compressor (10);
- an air pipe (9) to propel the solution to be tested into the nozzle (7) through the vaporizer head (6) ;
- a vaporization chamber (1) and a chamber cover (4); and
- a support (2) in the chamber (1).

2. The *in-vitro* airways nebulization assay according to claim 1, wherein said support (2) is a 6 to 48- well plate, preferentially a 12-well plate to 24-well plate or any other plate compatible with nebulization.

3. Use of an in-vitro airways nebulizing assay, according to claim 1 or 2, for evaluating products effects on airways pathologies and particularly on inflammation and/or inflammatory processes.

4. Use according to claim 3, for evaluating products effects, comprising the steps of:
a) from a sample of airway tissue and isolated cells or using 3D reconstructed airways tissue, culturing the cells under appropriate conditions able to provide at least airways cells layer;
b) placing a sample of airways epithelium upon a support (2) in a chamber (1);
c) switching on the nebulizing system;
d) applying a treatment by nebulization;
e) measuring the mRNA expression levels of at least one inflammatory interleukin;
f) comparing the values with standard expression levels of same inflammatory interleukin mRNA expression previously determined without treatment;
g) the modulation of inflammatory interleukin mRNA expression is provided when inflammatory interleukin mRNA expression level with product application is increased more than two (2) folds or decreased more than 20% in comparison with inflammatory interleukin mRNA expression level without product application and formulate a conclusion.

5. Use according to claim 4, wherein at least one inflammatory interleukin is selected from: Interleukin 1 (IL-1), Interleukin 5 (IL-5), Interleukin 6 (IL-6), Interleukin 8 (IL-8), Interleukin 13 (IL-13), Interleukin 17 (IL-17) and TNF-alpha.

6. Use according to claim 5, wherein at least one inflammatory interleukin is selected from: Interleukin 6 (IL-6), Interleukin 8 (IL-8) and TNF-alpha.

7. Use according to claim 6, wherein in step e) the mRNA expression level of at least IL-8 is measured and compare in step f) to values with standard expression levels of same IL-8 mRNA expression previously determined without treatment.

8. Use according to claim 6, wherein in step g) the modulation of IL-8 mRNA expression is provided when IL-8 mRNA expression level with product application is increased more than two (2) folds or decreased more than 20% in comparison with IL-8 mRNA expression level without product administration.

9. Use according to claim 4, wherein in step e) the inflammatory interleukin mRNA expression level of at least is measured by method selected from selected optical density, RT-PCR or any other applicable method.

10. Use according to claim 4, wherein sample of airways epithelium is within a medium.

11. Use according to claim 4, wherein the medium of airways epithelium is initially without Hydrocortisone.

## Patentansprüche

1. In-vitro-Atemwegsvernebelungsassay, umfassend mindestens:
- ein Vernebelungssystem mit einer Vernebelungseinrichtung; und
- ein 3D-rekonstruiertes Atemwegsepithel.
wobei das Vernebelungssystem mindestens umfasst:
- einen Vernebelungsbehälter (8);
- einen Kompressor (10);
- eine Luftleitung (9), um die zu testende Lösung durch den Verdampferkopf (6) in die Düse (7) zu befördern;
- eine Verdampfungskammer (1) und eine Kammerabdeckung (4); und
- einen Träger (2) in der Kammer (1).

2. In-vitro-Atemwegsvernebelungsassay nach Anspruch 1, wobei der Träger (2) eine Platte mit 6 bis 48 Vertiefungen, vorzugsweise eine Platte mit 12 bis 24 Vertiefungen oder eine beliebige andere mit der Vernebelung kompatible Platte ist.

3. Verwendung eines In-vitro-Atemwegsvernebelungsassays nach Anspruch 1 oder 2 zum Bewerten von Produktwirkungen auf Atemwegserkrankungen und insbesondere auf Entzündungen und/oder Entzündungsprozesse.

4. Verwendung nach Anspruch 3 zum Bewerten der Produktwirkungen, umfassend die Schritte:
a) Kultivieren der Zellen unter geeigneten Bedingungen, die in der Lage sind, mindestens eine Atemwegszellenschicht bereitzustellen, aus einer Probe von Atemwegsgewebe und isolierten Zellen oder unter Verwendung von 3D-rekonstruiertem Atemwegsgewebe;
b) Platzieren einer Probe von Atemwegsepithel auf einem Träger (2) in einer Kammer (1);
c) Einschalten des Vernebelungssystems;
d) Anwenden einer Behandlung durch Vernebelung;
e) Messen der mRNA-Expressionsgrade von mindestens einem entzündlichen Interleukin;
f) Vergleichen der Werte mit Standardexpressionsgraden derselben mRNA-Expression von entzündlichem Interleukin, die zuvor ohne Behandlung bestimmt wurde;
g) wobei die Modulation der mRNA-Expression von entzündlichem Interleukin bereitgestellt ist, wenn der mRNA-Expressionsgrad von entzündlichem Interleukin bei Produktanwendung im Vergleich mit dem mRNA-Expressionsgrad von entzündlichem Interleukin ohne Produktanwendung um mehr als den Faktor zwei (2) erhöht oder um mehr als 20 % verringert wird, und Formulieren einer Schlussfolgerung.

5. Verwendung nach Anspruch 4, wobei mindestens ein entzündliches Interleukin ausgewählt ist aus: Interleukin 1 (IL-1), Interleukin 5 (IL-5), Interleukin 6 (IL-6), Interleukin 8 (IL-8), Interleukin 13 (IL-13), Interleukin 17 (IL-17) und TNF-alpha.

6. Verwendung nach Anspruch 5, wobei mindestens ein entzündliches Interleukin ausgewählt ist aus: Interleukin 6 (IL-6), Interleukin 8 (IL-8) und TNF-alpha.

7. Verwendung nach Anspruch 6, wobei in Schritt e) der mRNA-Expressionsgrad von mindestens IL-8 gemessen wird und in Schritt f) mit Werten mit Standardexpressionsgraden derselben IL-8-mRNA-Expression verglichen wird, die zuvor ohne Behandlung bestimmt wurden.

8. Verwendung nach Anspruch 6, wobei in Schritt g) die Modulation der IL-8-mRNA-Expression bereitgestellt wird, wenn der IL-8-mRNA-Expressionsgrad bei Produktanwendung im Vergleich mit dem IL-8-mRNA-Expressionsgrad ohne Produktverabreichung um mehr als den Faktor zwei (2) erhöht oder um mehr als 20 % verringert wird.

9. Verwendung nach Anspruch 4, wobei in Schritt e) der mRNA-Expressionsgrad von entzündlichem Interleukin von mindestens durch eine Methode gemessen wird, die aus ausgewählter optischer Dichte, RT-PCR oder einer beliebigen anderen anwendbaren Methode ausgewählt ist.

10. Verwendung nach Anspruch 4, wobei sich die Probe des Atemwegsepithels innerhalb eines Mediums befindet.

11. Verwendung nach Anspruch 4, wobei das Medium des Atemwegsepithels zunächst kein Hydrocortison enthält.

## Revendications

1. Essai *in vitro* de nébulisation de voies respiratoires comprenant au moins :
- un système de nébulisation avec un appareil nébuliseur ; et
- un épithélium de voies respiratoires reconstruit en 3D.
dans lequel le système de nébulisation comprend au moins :
- un réservoir de nébuliseur (8) ;
- un compresseur (10) ;
- un tuyau d'air (9) pour propulser la solution à tester dans la buse (7) à travers la tête de vaporisateur (6) ;
- une chambre de vaporisation (1) et un couvercle de chambre (4) ; et
- un support (2) dans la chambre (1).

2. Essai *in vitro* de nébulisation de voies respiratoires selon la revendication 1, dans lequel ledit support (2) est une plaque de 6 à 48 puits, préférentiellement une plaque de 12 puits à une plaque de 24 puits ou une quelconque autre plaque compatible avec une nébulisation.

3. Utilisation d'un essai in vitro de nébulisation de voies respiratoires, selon la revendication 1 ou 2, pour l'évaluation des effets de produits sur des pathologies de voies respiratoires et en particulier sur l'inflammation et/ou des processus inflammatoires.

4. Utilisation selon la revendication 3, pour l'évaluation d'effets de produits, comprenant les étapes de :
a) à partir d'un échantillon de tissu de voies respiratoires et de cellules isolées ou à l'aide d'un tissu de voies respiratoires reconstruit en 3D, cultiver les cellules dans des conditions appropriées aptes à fournir au moins une couche de cellules de voies respiratoires ;
b) placer un échantillon d'épithélium de voies respiratoires sur un support (2) dans une chambre (1) ;
c) activer le système de nébulisation ;
d) appliquer un traitement par nébulisation ;
e) mesurer les niveaux d'expression d'ARNm d'au moins une interleukine inflammatoire ;
f) comparer les valeurs à des niveaux d'expression standard d'une même expression d'ARNm d'interleukine inflammatoire précédemment déterminée sans traitement ;
g) la modulation d'expression d'ARNm d'interleukine inflammatoire est fournie lorsqu'un niveau d'expression d'ARNm d'interleukine inflammatoire avec application de produit est augmentée de plus de deux (2) fois ou diminué de plus de 20 % en comparaison avec un niveau d'expression d'ARNm d'interleukine inflammatoire sans application de produit, et formuler une conclusion.

5. Utilisation selon la revendication 4, dans laquelle au moins une interleukine inflammatoire est choisie parmi : Interleukine 1 (IL-1),
Interleukine 5 (IL-5), Interleukine 6 (IL-6), Interleukine 8 (IL-8), Interleukine 13 (IL-13), Interleukine 17 (IL-17) et TNF-alpha.

6. Utilisation selon la revendication 5, dans laquelle au moins une interleukine inflammatoire est choisie parmi : Interleukine 6 (IL-6),
Interleukine 8 (IL-8) et TNF-alpha.

7. Utilisation selon la revendication 6, dans laquelle à l'étape e) le niveau d'expression d'ARNm d'au moins IL-8 est mesuré et comparé à l'étape f) à des valeurs avec des niveaux d'expression standard de même expression d'ARNm d'IL-8 précédemment déterminés sans traitement.

8. Utilisation selon la revendication 6, dans laquelle à l'étape g) la modulation d'expression d'ARNm d'IL-8 est fournie lorsqu'un niveau d'expression d'ARNm d'IL-8 avec application de produit est augmenté de plus de deux (2) fois ou diminué de plus de 20 % en comparaison avec un niveau d'expression d'ARNm d'IL-8 sans administration de produit.

9. Utilisation selon la revendication 4, dans laquelle à l'étape e) le niveau d'expression d'ARNm d'interleukine inflammatoire d'au moins est mesuré par un procédé choisi parmi la densité optique sélectionnée, la RT-PCR ou un quelconque autre procédé applicable.

10. Utilisation selon la revendication 4, dans laquelle l'échantillon d'épithélium de voies respiratoires est au sein d'un milieu.

11. Utilisation selon la revendication 4, dans laquelle le milieu d'épithélium de voies respiratoires est initialement dépourvu d'hydrocortisone.
